# EUROPÄISCHE PATENTSCHRIFT

(11) **EP 0 586 445 B1**
(45) Veröffentlichungstag und Bekanntmachung des Hinweises auf die Patenterteilung: **10.03.1999**
(21) Anmeldenummer: 92910495.8
(22) Anmeldetag: 20.05.1992
(51) Int. Cl.: C12P 21/08, C12N 5/20, G01N 33/574, G01N 33/577, C12N 15/06, C12N 15/12

(54) **MONOKLONALE ANTIKÖRPER GEGEN C-KIT**
MONOCLONAL ANTIBODIES AGAINST C-KIT
ANTICORPS MONOCLONAUX DIRIGES CONTRE C-KIT

(30) Priorität: 25.05.1991 DE 4117090; 20.02.1992 DE 4205148
(43) Veröffentlichungstag der Anmeldung: 16.03.1994
(73) Patentinhaber: BOEHRINGER MANNHEIM GMBH, 68298 Mannheim (DE); Max-Planck-Gesellschaft zur Förderung der Wissenschaften e.V., D-37073 Göttingen (DE)
(72) Erfinder: BARTKE, Ilse, D-8132 Tutzing (DE); KOSTKA, Günter, D-8000 München 40 (DE); NAUJOKS, Kurt, D-8122 Penzberg (DE); ULLRICH, Axel, D-8000 München 40 (DE)
(74) Vertreter: Fouquet, Herbert, Dr.
(86) Internationale Anmeldenummer: EP9201117
(87) Internationale Veröffentlichungsnummer: WO9221766

(56) Entgegenhaltungen:
- WO-A-89/01973
- EMBO JOURNAL Bd. 6, Nr. 11, November 1987, OXFORD, GB Seiten 3341 - 3351; Y. YARDEN ET AL.: 'Human proto-oncogene c-kit: a new cell surface receptor tyrosine kinase for an unidentified ligand.' in der Anmeldung erwähnt
- JOURNAL OF CELLULAR BIOCHEMISTRY Bd. SUP 0, Nr. 15F, 1991, NEW YORK, USA Seite 89; L. ASHMAN: 'A murine monoclonal antibody to the human c-kit proto-oncogene product SCF receptor effect on the growth of hemopoietic ürogenitor cells in-vitro and expression of c-kit in AML.'
- LEUKEMIA RESEARCH Bd. 14, Nr. 7, 1990, Seiten 637 - 644; L. ASHMAN ET AL.: 'A monoclonal antibody that inhibits the action of GM-CSF on normal but not leukaemic progenitors.'
- CLINICAL RESEARCH Bd. 39, Nr. 2, 1991, Seite 210A; V. BROUDY ET AL.: 'Isolation and characterization of a monoclonal antibody SR-1 that recognizes the stem cell factor receptor c-kit.'

## Beschreibung

Die Erfindung betrifft monoklonale Antikörper gegen den humanen c-kit-Rezeptor sowie ein Verfahren zum Nachweis der Malignität von Tumoren der hämatopoietischen Zellen, von Seminomen oder kleinzelligem Lungenkarzinom über den Nachweis einer veränderten Expression des c-kit-Rezeptors.

Entwicklung, Differenzierung und Stoffwechselfunktionen der Zellen eines mehrzelligen Organismus werden durch Hormone und Wachstumsfaktoren reguliert. Der erste Schritt ist in vielen Fällen die Bindung dieser Faktoren an einen Rezeptor an der Zelloberfläche. Diese Bindung löst bei einer Reihe dieser Rezeptoren die Phosphorylierung von Tyrosinresten verschiedener Zellproteine aus. Für diese Rezeptor-Tyrosinkinasen konnte ein entscheidender Einfluß auf das Wachstumsverhalten von Zellen nachgewiesen werden (Yarden und Ullrich, Ann. Rev. Biochem. 57 (1988), 443 - 478). Autokrine Regelsysteme unter Beteiligung von Rezeptor-Tyrosinkinasen spielen eine wichtige Rolle bei der Transformation von Zellinien, die als Tumor-Modellsystem dienen, sowie auch in primärem Tumorgewebe. Für bestimmte Rezeptor-Tyrosinkinasen, wie z.B. den EGF-Rezeptor und den her2/neu-Rezeptor, konnte ein direkter Zusammenhang zwischen der Überexpression des Rezeptors und der Aggressivität des Tumorgeschehens bewiesen werden (Slamon et.al., Science 244, 1989, 707 - 712; Di Fiore et.al., Cell 51, 1987, 1063 - 1070).

Ein weiteres Mitglied der Rezeptor-Tyrosinkinasen-Familie ist c-kit, das als zelluläres Homolog des viralen Onkogens v-kit (felines Leukämie-Virus HZ4-FeSV) entdeckt, kloniert und sequenziert wurde (Yarden et.al. EMBO-Journal 6 (1987), 3341 - 3351). Es weist eine weitgehende Homologie mit dem PDGF-Rezeptor und dem CSF-1-Rezeptor (c-fms) auf. Untersuchungen mit Hilfe der Northern-Blot-Technik weisen auf eine charakteristische Verteilung des Rezeptors in den unterschiedlichen Geweben hin. Eine hohe Expression des c-kit-Gens konnte in hämatopoietischen Zellen und vor allem in Hirngeweben, Melanoblasten, Ovarien und in Stammzellen der Testis nachgewiesen werden (Orr-Urtreger et.al., Developement 109 (1990), 911 - 923). Untersuchungen an verschiedenen erythroiden und myeloiden Zellinien weisen auf eine Expression des c-kit-Gens in frühen Differenzierungsstufen hin (André et.al., Oncogene 4 (1989), 1047 - 1049). Bestimmte Tumoren, wie z.B. Glioblastomazellen zeigen ebenfalls eine deutliche Expression des c-kit-Gens.

Als natürlicher Ligand des c-kit Rezeptors wurde inzwischen ein etwa 30kD großes Protein aus dem Kulturüberstand von murinen Fibroblasten isoliert und als MGF = mast cell growthfactor (Williams et.al., Cell 63 (1990), 167 - 174) oder hämatopoietischer Wachstumsfaktor KL (Huang et.al., Cell 63, (1990), 225 - 233) bezeichnet. Ein entsprechendes Protein wurde auch aus Rattenleberzellen isoliert und SCF = stem cell factor genannt (Zsebo et.al., Cell 63 (1990), 195 - 201). Der entsprechende humane Faktor wurde von Martin et al. kloniert und wird synonym als SCF, MGF oder Steel Factor (SF) bezeichnet (Cell 63 (1990), 203-211).

Die Expression des c-kit-Gens wurde bislang nur auf der Ebene der Transkription über den Nachweis der c-kit-mRNA, insbesondere mit Hilfe der Northern-Blot-Technik bestimmt. Dieses Verfahren kann jedoch nur indirekt Hinweise auf die Menge des exprimierten Proteins geben, da z.B. Regulationsvorgänge auf Translationsebene oder die Stabilität des gebildeten c-kit-Rezeptor-Proteins hierbei überhaupt nicht erfaßt werden. Für die Diagnostik ist es daher von entscheidender Bedeutung, direkt die Menge des exprimierten Proteins zu bestimmen. Dies ist mit der erforderlichen Spezifität der Aussage nur mit Hilfe monoklonaler Antikörper gegen den c-kit-Rezeptor möglich.

Von N. Lerner et.al. (Blood 77 (1991), 1876-1883) wurde gezeigt, daß der monoklonale Antikörper YB5.B8 von Gadd et.al. (Leuk.Res. 9 (1985), 1329), der gegen leukämische Blasten eines Patienten mit akuter myeloischer Leukämie gerichtet ist, wahrscheinlich mit dem c-kit Protein reagiert. Ein monoklonaler Antikörper gegen den c-kit-Rezeptor kann jedoch mit eindeutiger Sicherheit nur durch Immunisierung mit einem definierten c-kit Antigen erhalten werden.

Es war daher die Aufgabe der Erfindung, monoklonale Antikörper gegen den c-kit-Rezeptor sowie ein Verfahren zu deren Herstellung zur Verfügung zu stellen, sowie ein Verfahren zur Verfügung zu stellen, das es ermöglicht, mit Hilfe dieser Antikörper eine veränderte Expression dieses c-kit-Rezeptors nachzuweisen.

Diese Aufgabe wird gelöst durch monoklonale Antikörper gegen den humanen c-kit-Rezeptor, die aus den Zellinien DSM ACC 2007 oder DSM ACC 2009 erhältlich sind oder in äquivalenter Weise an den c-kit-Rezeptor bindefähig sind, wie die von den Zellinien DSM ACC 2007 oder DSM ACC 2009 produzierten Antikörper.

Unter dem Begriff "in äquivalenter Weise bindefähiger Antikörper" werden Antikörper verstanden, bei denen eine Epitopüberlappung mit den Antikörpern die aus den Zellinien DSM ACC 2007 oder DSM 2009 erhältlich sind nachweisbar ist. Diese Epitopüberlappung kann mit Hilfe eines kompetitiven Testsystems leicht nachgewiesen werden. Dazu wird zum Beispiel mit Hilfe eines Enzym-Immunoassays überprüft, inwieweit ein Antikörper mit dem bekannten Antikörper um die Bindung an ein definiertes Antigen bzw. ein spezielles Epitop konkurriert. Dazu inkubiert man das entsprechende Antigen mit dem bekannten monoklonalen Antikörper in markierter Form und einem Überschuß des in Betracht gezogenen Antikörpers. Durch Immobilisierung der gebildeten Komplexe, Trennung der festen von der flüssigen Phase und Nachweis der gebundenen Markierung in einer der beiden Phasen kann dann leicht festgestellt werden, inwieweit der in Betracht gezogene Antikörper den definierten Antikörper aus der Bindung verdrängen kann. Ist eine Verdrängung von mindestens 50 % bei 10⁵-fachem Überschuß gegeben, so liegt eine Epitopüberlappung vor.

Es hat sich überraschenderweise gezeigt, daß die erhaltenen Antikörper spezifisch nur mit Seminomen und kleinzelligem Lungenkarzinom (DSM ACC 2007 und DSM ACC 2009) reagieren, nicht aber mit gesundem Lungengewebe.

Es hat sich weiterhin überraschenderweise gezeigt, daß der monoklonale Antikörper DSM ACC 2007 die Stimulierung der Proliferation lymphoider Zellen durch den Stammzellfaktor SCF inhibiert. Dieser Antikörper hemmt zudem auch die Bindung von Wachstumsfaktoren wie SCF an den c-kit-Rezeptor und damit auch die SCF-induzierte Phosphorylierung des c-kit-Rezeptors, sowie die SCF induzierte Abnahme der Expression von c-kit-Rezeptormolekülen.

Ein weiterer Gegenstand der Erfindung ist daher ein monoklonaler Antikörper gegen den humanen c-kit-Rezeptor, welcher die Bindung von Wachstumsfaktoren an den c-kit-Rezeptor inhibiert und erhältlich ist durch Immunisierung mit Säugerzellen, die mit einer in diesen Zellen exprimierbaren c-kit-Sequenz transfiziert wurden, Immortalisierung der Milzzellen der immunisierten Tiere, Identifizierung derjenigen Hybridomzellen, die den gewünschten Antikörper produzieren, über die Bestimmung der Bindung des erhaltenen Antikörpers an den c-kit-Rezeptor, sowie die Bestimmung der Wirkung dieses Antikörpers auf die Bindung von Wachstumsfaktoren an den c-kit-Rezeptor, Identifizierung solcher Hybridomzellen, deren Kulturüberstand dabei eine hemmende Wirkung aufweist, Klonierung dieser Hybridomzellen und Isolierung des von diesen Klonen produzierten Antikörpers nach bekannten Verfahren.

Ein bevorzugter Gegenstand der Erfindung ist ein monoklonaler Antikörper gegen den humanen c-kit-Rezeptor, welcher die Stimulierung der Proliferation lymphoider Zellen durch SCF inhibiert und erhältlich ist durch Immunisierung mit Säugerzellen, die mit einer in diesen Zellen exprimierbaren c-kit-Sequenz transfiziert wurden, Immortalisierung der Milzzellen der immunisierten Tiere, Identifizierung derjenigen Hybridomzellen, die den gewünschten Antikörper produzieren, über die Bestimmung der Bindung des erhaltenen Antikörpers an den c-kit-Rezeptor, sowie die Bestimmung der Wirkung dieses Antikörpers auf die Proliferation lymphoider Zellen, Identifizierung solcher Hybridomzellen, deren Kulturüberstand dabei eine hemmende Wirkung aufweist, Klonierung dieser Hybridomzellen und Isolierung des von diesen Klonen produzierten Antikörpers nach bekannten Verfahren.

Die Immunisierung wird in den hierfür üblicherweise verwendeten Tieren, wie z.B. Mäusen oder Ratten durchgeführt. Vorzugsweise werden Mäuse verwendet.

Als Immunogen werden vorzugsweise NIH-3T3 Zellen verwendet, die mit einer für das c-kit Protein codierenden cDNA transfiziert wurden. Die Klonierung dieser cDNA wurde wie von Yarden et. al., (EMBO J. 6 (1987), 3341 - 3351) beschrieben, durchgeführt. Die für die Immunisierung verwendete transfizierte NIH-3T3 Zellinie c-kit wurde am 23.05.1991 bei der Deutschen Sammlung von Mikroorganismen und Zellkulturen GmbH unter der Hinterlegungsnummer DSM ACC 2006 hinterlegt.

Die Immortalisierung der Milzzellen der immunisierten Tiere erfolgt vorzugsweise durch Fusionierung mit der Myelomzellinie P3X63-Ag8.653 (ATCC CRL 1580) gemäß der Methode nach J.of.Imm. Meth. 39 (1980) 285 - 308. Daneben können aber auch andere, dem Fachmann geläufige Verfahren zur Immortalisierung der Milzzellen (z.B. EBV-Transformation) verwendet werden.

Zur Klonierung werden die Zellen z.B. mittels eines Fluoreszenz-aktivierten Zellsorters vereinzelt. Zum Nachweis von immortalisierten Zellen, die den gewünschten Antikörper gegen c-kit produzieren, wird eine Probe des Kulturüberstandes in einem ELISA-Test auf Reaktivität mit dem c-kit-Rezeptor getestet. Um solche Antikörper zu erhalten, welche die Bindung von Wachstumsfaktoren an den c-kit-Rezeptor bzw. die Stimulierung der Proliferation lymphoider Zellen durch SCF inhibieren, wird der Kulturüberstand derjenigen Klone, die an den c-kit-Rezeptor bindende Antikörper produzieren, zusätzlich auf die Hemmung der Bindung von Wachstumsfaktoren an den c-kit-Rezeptor bzw. die Hemmung der SCF stimulierten Proliferaton lymphoider Zellen untersucht.

Die Hemmung der Bindung von Wachstumsfaktoren an den c-kit-Rezeptor ergibt sich in üblicher Weise durch Inkubation eines markierten Wachstumsfaktors mit dem c-kit-Rezeptor in Gegenwart des zu untersuchenden Antikörpers und anschließender Bestimmung von Rezeptor-gebundenem Wachstumtsfaktor.

Die Hemmung der SCF induzierten Proliferation lymphoider Zellen wird in üblicher Weise bestimmt, vorzugsweise über die Fluoreszenzmarkierung von Lymphozyten, deren Proliferation mit SCF stimuliert wurde.

Diejenigen Klone, deren Kulturüberstand eine positive Reaktion ergibt, werden expandiert und die von diesen Klonen produzierten Antikörper nach bekannten Verfahren isoliert.

Es hat sich überraschenderweise gezeigt, daß der Antikörper DSM ACC 2007 an den mit Paraffin behandelten c-kit-Rezeptor ebenso bindet wie an den nicht mit Paraffin behandelten c-kit-Rezeptor.

In einer bevorzugten Ausführungsform der Erfindung werden solche Hybridomzellen kloniert, deren Kulturüberstand solche Antikörper enthält, die auch mit dem paraffin-behandelten c-kit-Rezeptor reagieren und die von diesen Klonen produzierten Antikörper nach bekannten Verfahren isoliert.

Der Nachweis von immortalisierten Zellen, die den gewünschten Antikörper gegen c-kit produzieren, erfolgt bei dieser bevorzugten Ausführungsform über die Inkubation einer Probe des Kulturüberstandes mit einem paraffin-behandelten Gewebeschnitt und Nachweis des an den in diesem Gewebe schnitt enthaltenen c-kit-Rezeptor gebundenen Antikörpers.

Der monoklonale Antikörper DSM ACC 2009 fördert überraschenderweise synergistisch die durch den Stammzellfaktor SCF induzierte Proliferation lymphoider Zellen. Er stimuliert zudem die SCF induzierte Phosphorylierung des c-kit-Rezeptors und bewirkt eine geringe Verzögerung der SCF induzierten Abnahme der c-kit-Rezeptor Expression.

Ein weiterer Gegenstand der Erfindung ist daher ein monoklonaler Antikörper gegen den humanen c-kit-Rezeptor, welcher die SCF induzierte Proliferation lymphoider Zellen stimuliert und erhältlich ist durch Immunisierung mit Säugerzellen, die mit einer in diesen Zellen exprimierbaren c-kit-Sequenz transfiziert wurden, Immortalisierung der Milzzellen der immunisierten Tiere, Identifizierung derjenigen Hybridomzellen, die den gewünschten Antikörper produzieren, über die Bestimmung der Bindung des erhaltenen Antikörpers an den c-kit-Rezeptor, sowie die Bestimmung der Wirkung dieses Antikörpers auf die Proliferation lymphoider Zellen, Identifizierung solcher Hybridomzellen, deren Kulturüberstand dabei eine stimulierende Wirkung aufweist, Klonierung dieser Hybridomzellen und Isolierung des von diesen Klonen produzierten Antikörpers nach bekannten Verfahren.

Die Stimulierung der SCF induzierten Proliferation lymphoider Zellen wird in üblicher Weise bestimmt, vorzugsweise über Fluoreszenzmarkierung von Lymphozyten, deren Proliferation mit SCF stimuliert wurde.

Ein weiterer Gegenstand der Erfindung ist ein Verfahren zur Herstellung von monoklonalen Antikörpern gegen den humanen c-kit-Rezeptor durch Immunisierung mit Säugerzellen, die mit einer in diesen Zellen exprimierbaren c-kit-Sequenz transfiziert wurden und Immortalisierung der Milzzellen der immunisierten Tiere, wobei man solche Hybridomzellen kloniert, die Antikörper gegen den humanen c-kit-Rezeptor produzieren und die von diesen Klonen produzierten Antikörper nach bekannten Verfahren isoliert.

Ein besonders bevorzugter Gegenstand der Erfindung ist ein Verfahren zur Herstellung von monoklonalen Antikörpern gegen den humanen c-kit-Rezeptor, welche die Stimulierung der Proliferation lymphoider Zellen durch SCF inhibieren und erhältlich sind durch Immunisierung mit Säugerzellen, die mit einer in diesen Zellen exprimierbaren c-kit-Sequenz transfiziert wurden, Immortalisierung der Milzzellen der immunisierten Tiere, Identifizierung derjenigen Hybridomzellen, die den gewünschten Antikörper produzieren, über die Bestimmung der Bindung des erhaltenen Antikörpers an den c-kit-Rezeptor, sowie die Bestimmung der Wirkung dieses Antikörpers auf die Proliferation lymphoider Zellen, Identifizierung solcher Hybridomzellen, deren Kulturüberstand dabei eine hemmende Wirkung aufweist, Klonierung dieser Hybridomzellen und Isolierung der von diesen Klonen produzierten Antikörper nach bekannten Verfahren.

Ein besonders bevorzugter Gegenstand der Erfindung ist weiterhin ein Verfahren zur Herstellung von monoklonalen Antikörpern gegen den humanen c-kit-Rezeptor, welche die SCF induzierte der Proliferation lymphoider Zellen stimulieren und erhältlich sind durch Immunisierung mit Säugerzellen, die mit einer in diesen Zellen exprimierbaren c-kit-Sequenz transfiziert wurden und Immortalisierung der Milzzellen der immunisierten Tiere, Identifizierung derjenigen Hybridomzellen, die den gewünschten Antikörper produzieren, über die Bestimmung der Bindung des erhaltenen Antikörpers an den c-kit-Rezeptor, sowie die Bestimmung der Wirkung dieses Antikörpers auf die Proliferation lymphoider Zellen, Identifizierung solcher Hybridomzellen, deren Kulturüberstand dabei eine stimulierende Wirkung aufweist, Klonierung dieser Hybridomzellen und Isolierung der von diesen Klonen produzierten Antikörper nach bekannten Verfahren.

Weiterhin sind Gegenstand der Erfindung die Zellinien DSM ACC 2007 und DSM ACC 2009.

Es hat sich überraschenderweise gezeigt, daß mit den erfindungsgemäßen monoklonalen Antikörpern gegen c-kit eine Beurteilung der Malignität von Tumoren im Bereich der hämatopoietischen Zellen, von Seminomen sowie von kleinzelligem Lungenkarzinom erreicht werden kann, die wertvolle Hinweise für eine Therapie liefert.

Ein weiterer Gegenstand der Erfindung ist daher ein Verfahren zum Nachweis der Malignität von Tumoren der hämatopoietischen Zellen, von Seminomen und kleinzelligem Lungenkarzinom bei dem man eine Gewebeprobe mit mindestens einem monoklonalen Antikörper gegen den c-kit-Rezeptor inkubiert und anschließend gebundene Antikörper mittels bekannter Methoden nachweist.

In einer bevorzugten Ausführungsform wird die Gewebeprobe mit einem erfindungsgemäßen Antikörper gegen den c-kit-Rezeptor inkubiert. Vorzugsweise wird dabei ein Antikörper vom IgG-Isotyp verwendet. Es können aber auch Fab oder F(ab')₂ Fragmente verwendet werden.

Zum Nachweis von gebundenem Antikörper gegen den c-kit-Rezeptor wird beispielsweise mit einem vor oder nach der Bindung an den c-kit-Antikörper markierten zweiten Antikörper gegen murines Fcγ inkubiert. Als Markierung wird üblicherweise ein Enzym, ein Floureszenz- oder Chemilumineszenzfarbstoff verwendet.

Maligne Tumoren der hämatopoietischen Zellen, maligne Seminome sowie bei Verwendung von monoklonalen Antikörpern, die in äquivalenter Weise wie die von den Zellinien DSM ACC 2007, oder DSM ACC 2009 produzierten Antikörper an den c-kit-Rezeptor binden, auch kleinzellige Lungenkarzinome zeigen dabei ein positives Signal.

Eine weitere Verwendung von erfindungsgemäßen monoklonalen Antikörpers gegen den humanen c-kit-Rezeptor, welche die Stimulierung der Proliferation lymphoider Zellen durch SCF inhibieren, ist die Verwendung zur Herstellung eines Therapeutikums zur Behandlung einer myeloischen Leukämie.

Die erfindungsgemäßen Zellinien DSM ACC 2006, DSM ACC 2007 und DSM ACC 2009 wurden am 23.05.1991 bei der Deutschen Sammlung von Mikroorganismen und Zellkulturen GmbH, Mascheroder Weg 1b, D-3300 Braunschweig hinterlegt.

Die Erfindung wird durch folgende Beispiele in Verbindung mit der Abbildung erläutert.

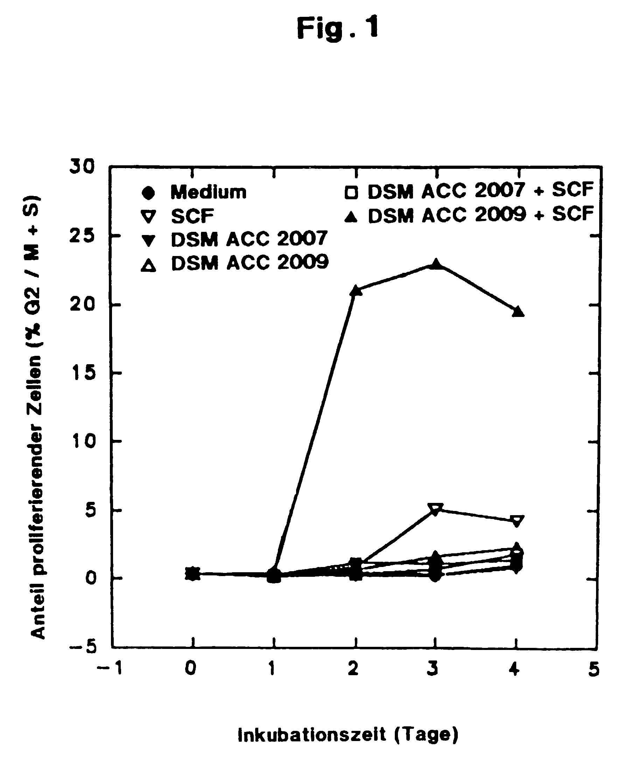

Figur 1 zeigt die Stimulierung der SCF-induzierten Proliferation lymphoider Zellen durch den monoklonalen Antikörper DSM ACC 2009, sowie die Hemmung der SCF-induzierten Proliferation lymphoider Zellen durch den monoklonalen Antikörper DSM ACC 2007. Gezeigt wird jeweils der Anteil proliferierender Zellen nach Inkubation von AML-Zellen bei 37°C mit Medium (•), 100 µg/ml SCF (∇), 2 µg/ml der monoklonalen Antikörper DSM ACC 2007 (▼) bzw. DSM ACC 2009 (Δ), sowie nach Vorinkubation mit den monoklonalen Antikörpern DSM ACC 2007 (□) bzw. DSM ACC 2009 (▲) für 30 Minuten bei 4°C vor der Stimulierung mit SCF.

### Beispiel 1

### Gewinnung monoklonaler Antikörper gegen den humanen c-kit-Rezeptor

Das humane c-kit-Gen wird wie von Yarden et. al., (EMBO-Journal, volume 6 (1987), 3341 - 3351) beschrieben, kloniert und in NIH-3T3-Zellen exprimiert. Mit 5.000.000 dieser transfizierten, c-kit-exprimierenden NIH-3T3-Zellen werden Balb/c-Mäuse intraperitoneal immunisiert. Diese Immunisierung wird in ca. 4-wöchigen Abständen wiederholt. Die Immunisierungsdauer beträgt insgesamt 6 Monate. Anschließend werden die Tiere noch zweimal mit wheat germ agglutinin angereichertem c-kit (s. Beispiel 2) immunisiert, die letzte Immunisierung wird dabei intravenös durchgeführt. Drei Tage nach dieser letzten Immunisierung erfolgt die Immortalisierung von Milzzellen der immunisierten Tiere mit der Myelomzellinie P3X63-Ag8.653 (ATCC CRL 1580).

Die Fusion der Milzzellen mit der Myelomzellinie wird nach dem Standardverfahren gemäß J. of Imm. Meth., 39 (1980), 285 - 308 durchgeführt. Das Fusionsverhältnis Milzzellen:Myelomzellen ist dabei 1:1. Die Fusionsprodukte werden auf 24er Kulturschalen der Firma Greiner mit 5x10⁴ Peritonealexsudatzellen der Maus ausgesät. Positive Primärkulturen (Bestimmung gemäß Beispiel 3) werden zwei Wochen nach Fusion mit Hilfe eines Floureszenz-aktivierten Zellsorters (Becton Dickinson) kloniert. Die Zellen werden dabei einzeln in 96er Mikrotiterplatten abgelegt und mit HECS-Medium (Tecnomara) gefüttert. Als Kulturmedium wird anschließend handelsübliches RPMI-1640-Medium mit 10%igem fötalem Kälberserum verwendet.

Zur Gewinnung der monoklonalen Antikörper werden die so erhaltenen Hybridom-Zellklone in vivo expandiert. Dazu werden 5 x 10⁶ Hybridom Zellen intraperitoneal in mit Pristan (Sigma) vorbehandelte Mäuse inokkultiert. Nach 10 - 15 Tagen werden je Maus 2 - 3 ml Ascites entnommen und daraus der monoklonale Antikörper nach herkömmlichen Methoden gewonnen. Die Ausbeute beträgt etwa 5 mg IgG/ml Ascites.

### Beispiel 2

### Anreicherung des c-kit-Rezeptor-Proteins über wheat germ agglutinin Agarose

Die c-kit exprimierende transfizierte NIH-3T3 Zellinie c-kit wird in DMEM/F12 (1:1) Medium, das 10 % FKS und 2 mmol/l Glutamin enthält, bis zur Konfluenz in Rollerflaschen kultiviert. Anschließend werden die Zellen in 20 ml Lysepuffer lysiert. Der Lysepuffer enthält 50 mmol/l Hepes pH 7,5; 150 mmol/l Natriumchlorid; 1,5 mmol/1 MgCl₂; 1mmol/l EGTA; 1 % Triton X-100; 10 % Glycerin und 4 µg/ml PMSF. Das Lysat wird 1 Stunde bei 100.000 g zentrifugiert, der Überstand 1:1 mit Her2x-Puffer verdünnt und über Nacht bei 40°C im Batch-Verfahren an wheat germ agglutinin-Agarose (Sigma) gebunden (Her2x-Puffer: 50 mmol/l Hepes pH 7,2; 150 mmol/l Natriumchlorid; 1 % Triton X-100; 10 % Glycerin und 4 µg/ml PMSF). Anschließend wird die wheat germ agglutinin-Agarose mit 10 Volumen Her2x-Puffer gewaschen und schließlich c-kit-Rezeptor-Protein mit 0,3 mol/l N-Acetyl-glucosamin in Her2x-Puffer eluiert.

### Beispiel 3

### Bestimmung der Spezifität der produzierten Antikörper

Um die Spezifität der Antikörper im Kulturüberstand der Hybridomzellen zu erfassen, wird ein Enzyme-Linked Immuno-Sorbent-Assay (ELISA) angewendet.

Dazu werden 96er Mikrotiterplatten (Nunc) mit 100 µl c-kit-Antigen (Isolierung nach Beispiel 2; 5 µg/ml in Carbonatpuffer, Boehringer Mannheim, Katalog Nr. 726559) beschichtet, mit 100 µl Kulturüberstand (1:25 mit PBS (nach Dulbecco und Vogt, J.Exp.Med. 99 (1954), 167 - 182) verdünnt) 2 Stunden bei Raumtemperatur inkubiert und mit 3 x 350 µl PBS/0,05% Tween 20 gewaschen. Danach wird mit POD markiertem Schaf-Anti-Maus IgG (10 mU; Boehringer Mannheim, Katalog Nr. 1317377) 15 Minuten bei Raumtemperatur inkubiert, mit 3 x 350 µl PBS/0,05% Tween 20 gewaschen und die Nachweisreaktion mit 100 pl ABTS® (1 mg/ml, Boehringer Mannheim Katalog Nr. 756 407) in 40 mmol/l Citrat-Puffer pH 4,4, der 3,25 mmol/l Natriumperborat enthält (Boehringer Mannheim, Katalog Nr. 1204 530) ausgelöst. Nach 20 Minuten Inkubation bei Raumtemperatur werden die Extinktionen in einem Photometer bei 405 nm bestimmt.

### Beispiel 4

### Bestimmung der Wirkung der produzierten Antikörper auf die SCF induzierte Proliferation lymphoider Zellen und die Bindung von Wachstumsfaktoren

Lymphozyten werden durch Dichtegradientenzentrifugation (Lymphozyten-Trennmedium Boehringer Mannheim GmbH, Katalog-Nr. 295 949) isoliert und in RPMI 1640 komplett (10 % fötales Kälberserum, 2 mmol/l Glutamin, 1 % Vitaminlösung (Boehringer Mannheim GmbH, Katalog-Nr. 210 307), 100 IU/ml Penicillin und 100 µg/ml Streptomycin) auf einen Zelltiter von 1 x 10⁶ Zellen/ml eingestellt. 100 µl dieser Zellsuspension werden zusammen mit 10 µl rekombinantem menschlichen SCF (Amgen Thousand Oaks USA; Endkonzentration 100 ng/ml), sowie in Parallelansätzen mit 2 µg/ml des zu untersuchenden monoklonalen Antikörpers für 30 Minuten bei 37°C inkubiert. In zwei weiteren Parallelansätzen wird zunächst mit 2 µg/ml des zu untersuchenden monoklonalen Antikörpers für 30 Minuten bei 4°C vorinkubiert und anschließend 100 ng/ml SCF zugesetzt. Anschließend werden diejenigen Zellen, die einen zu untersuchenden monoklonalen Antikörper gebunden haben, durch Fluoreszenzmarkierung mit FITC gekoppeltem Anti-Maus-Immunglobulin aus dem Schaf angefärbt (Boehringer Mannheim GmbH, Katalog-Nr. 821 462), sowie die DNA dieser Zellen durch Behandlung der Zellen mit einer hypotonen Lösung von 0,05 mg/ml Propidiumjodid (Sigma) und 0,1 % Natriumcitrat angefärbt und die Markierungen in einem FACS IV Zellsorter (Becton Dickinson) untersucht. Die Anregung erfolgt bei 488 nm , die Messung der FITC-markierten Zellen bei 530 nm, und die der Propidiumjodid-markierten Kerne bei 610 nm. Der prozentuale Anteil von proliferierenden Zellen ergibt sich aus der Summe der mit Propidiumjodid-markierten Anteile von Zellen, die sich in der S-Phase, sowie in der G2- bzw. M-Phase befinden (%S + %G2/M). Die Figur 1 zeigt den stimulierenden Effekt des monoklonalen Antikörpers DSM ACC 2009 auf die SCF-induzierte Proliferation lymphoider Zellen, sowie den hemmenden Effekt des monoklonalen Antikörpers DSM ACC 2007 auf die SCF-induzierte Proliferation lymphoider Zellen.

Der monoklonale Antikörper DSM ACC 2007 bewirkt zusätzlich eine Hemmung der Bindung von SCF an den c-kit-Rezeptor. Zum Nachweis dieser Wirkung werden die wie oben beschriebenen isolierten Lymphozyten 30 Minuten auf Eis in PBS mit 0,01 % NaN₃ und 2 µg/ml des monoklonalen Antikörpers DSM ACC 2007 inkubiert. Anschließend werden 125 ng/ml biotinylierter SCF zugegeben und die Zellen mit Streptavidin-Phycoerythrin (Dianova) gefärbt. Die Auswertung erfolgt in einem FACS IV Cellsorter (Becton Dickinson) mit einer Anregungswellenlänge von 488 nm und einer Messung bei 570 nm. Im Vergleich zu einem Kontrollsatz ohne Inkubation mit dem monoklonalen Antikörper DSM ACC 2007 ergibt sich eine 98 %-ige Hemmung der SCF-Bindung an Lymphozyten durch den monoklonalen Antikörper DSM ACC 2007.

Medium (RPMI 1640 komplett)
440 ml RPMI 1640 (Boehringer Mannheim BM 209 945)
50 ml fötales Kälberserum (FKS) (BM 210 471)
5 ml Glutaminlösung, 200 mmol/l (BM 210 277)
5 ml Vitaminlösung (1 %) (BM 210 307)
1 ml Penicillin (50 000 IU) und Streptomycin (50 mg)

| Vitaminlösung (BM 210 307): | | | |
|---|---|---|---|
| | mg/100 ml | | mg/100 ml |
| Ca-D(+)-Pantothenat | 10,0 | Nicotinsäureamid | 10,0 |
| Cholinchlorid | 10,0 | Pyridoxal . HCl | 10,0 |
| Folsäure | 10,0 | Riboflavin | 1,0 |
| meso-Inosit | 20,0 | Thiamin . HCl | 10,0 |

### Beispiel 5

### Bestimmung der Epitopüberlappung von Antikörpern gegen c-kit

Zum Nachweis der Epitopüberlappung eines Antikörpers mit dem monoklonalen Antikörper DSM ACC 2007 wird ein kompetetiver Enzym-Immunoassay durchgeführt. Dazu wird das nach Beispiel 2 angereicherte c-kit-Rezeptor Protein zunächst mit D-Biotinyl-E-amidocaponsäure-N-hydroxysuccinimidester (Boehringer Mannheim, Katalog-Nr. 1008960) gemäß Angaben des Herstellers biotinyliert. Von diesem biotinylierten Antigen werden 300 ng in einem Volumen von 100 µl PBS durch einstündige Inkubation bei Raumtemperatur an eine mit Streptavidin beschichtete Mikrotiterplatte (Herstellung nach EP-A 0 344 578) gebunden. Nach viermaligem Waschen mit PBS/0,05 % Tween 20 wird 90 Minuten bei Raumtemperatur simultan inkubiert mit dem monoklonalen Antikörper DSM ACC 2007, der mit Peroxidase markiert wurde (Endkonzentration 250 mU/ml) und dem zu beurteilenden Antikörper. Nach erneutem viermaligem Waschen mit PBS/0,05 % Tween 20 wird mit der Enzymsubstratlösung ABTS® in Natriumperborat enthaltendem Puffer 30 Minuten bei Raumtemperatur inkubiert und anschließend die Extinktion bei 405 nm als Maß für die Menge des gebundenen, POD markierten, monoklonalen Antikörpers DSM ACC 2007 gemessen. Dieser Wert wurde verglichen mit der Extinktion, die erhalten wurde, bei Inkubation mit dem monoklonalen Antikörper DSM ACC 2007 allein. Wenn bis zu einem 10⁵-fachen Überschuß an zu beurteilendem Antikörper gegenüber dem monoklonalen Antikörper DSM ACC 2007 Enzymkonjugat (250 mU/ml) mindestens 50 % Kompetition zu erkennen sind, liegt eine Epitopüberlappung vor.

### Beispiel 6

### Bestimmung der Expression des c-kit-Rezeptor Proteins in verschiedenen Geweben und Tumoren

Der zu untersuchende Gewebeschnitt wird 2 Stunden bei 4°C mit einem monoklonalen Antikörper gegen c-kit (DSM ACC 2007, DSM ACC 2009 jeweils 20 µg/ml) inkubiert. Nach einem Waschschritt (3 x 5 Minuten in PBS/0,05% Tween-20) wird der Gewebeschnitt mit Schaf-Anti-Maus IgG (100 µg/ml, Boehringer Mannheim, Katalog Nr. 1092618) 1 Stunde bei Raumtemperatur inkubiert. Gebundener Antikörper wird über einen Komplex aus Peroxidase und murinem Anti-Peroxidase-Antikörper (250 mU/ml, Boehringer Mannheim, Katalog Nr. 1092 626, 1 Stunde Inkubation bei Raumtemperatur) nachgewiesen. Die Nachweisreaktion wird mit Diaminobenzidin (1 mg/ml) ausgelöst und an einem Mikroskop ausgewertet.

Die folgende Tabelle zeigt die nach dieser Methode bestimmte Reaktivität der aus den Zellinien DSM ACC 2007 und DSM ACC 2009 erhaltenen monoklonalen Antikörper.

| | monoklonale Antikörper | |
|---|---|---|
| Gewebe | DSM ACC 2007 | DSM ACC 2009 |
| Seminom | + | + |
| | | |
| kleinzelliges Lungenkarzinom | + | + |
| | | |
| gesundes Lungengewebe | - | - |

## Patentansprüche

1. Monoklonaler Antikörper gegen den humanen c-kit-Rezeptor, erhältlich aus den Zellinien DSM ACC 2007 oder DSM ACC 2009 oder der an das Epitop bindet, an das die von den Zellinien DSM ACC 2007 oder DSM ACC 2009 produzierten Antikörper binden.

2. Monoklonaler Antikörper nach Anspruch 1 gegen den humanen c-kit-Rezeptor, welcher die Stimulierung der Proliferation lymphoider Zellen durch SCF inhibiert und erhältlich ist durch Immunisierung mit Säugerzellen, die mit einer in diesen Zellen exprimierbaren c-kit-Sequenz transfiziert wurden, Immortalisierung der Milzzellen der immunisierten Tiere, Identifizierung derjenigen Hybridomzellen, die den gewünschten Antikörper produzieren, über die Bestimmung der Bindung des erhaltenen Antikörpers an den c-kit-Rezeptor, sowie die Bestimmung der Wirkung dieses Antikörpers auf die Proliferation lymphoider Zellen, Identifizierung solcher Hybridomzellen, deren Kulturüberstand dabei eine hemmende Wirkung aufweist, Klonierung dieser Hybridomzellen und Isolierung des von diesen Klonen produzierten Antikörpers nach bekannten Verfahren.

3. Monoklonaler Antikörper nach Anspruch 1 gegen den humanen c-kit-Rezeptor, welcher die SCF-induzierte Proliferation lymphoider Zellen stimuliert und erhältlich ist durch Immunisierung mit Säugerzellen, die mit einer in diesen Zellen exprimierbaren c-kit-Sequenz transfiziert wurden, Immortalisierung der Milzzellen der immunisierten Tiere, Identifizierung derjenigen Hybridomzellen, die den gewünschten Antikörper produzieren, über die Bestimmung der Bindung des erhaltenen Antikörpers an den c-kit-Rezeptor, sowie die Bestimmung der Wirkung dieses Antikörpers auf die Proliferation lymphoider Zellen, Klonierung der auf diese Weise identifizierten gewünschten Hybridomzellen und Isolierung des von diesen Klonen produzierten Antikörpers nach bekannten Verfahren.

4. Verfahren zur Herstellung monoklonaler Antikörper gegen den humanen c-kit-Rezeptor nach Anspruch 1 durch Immunisierung mit Säugerzellen, die mit einer in diesen Zellen exprimierbaren c-kit-Sequenz transfiziert wurden, Immortalisierung der Milzzellen der immun isierten Tiere, Klonierung von solchen Hybridomzellen, die Antikörper gegen den humanen c-kit-Rezeptor produzieren und Isolierung der von diesen Klonen produzierten Antikörper nach bekannten Verfahren.

5. Verfahren nach Anspruch 4, dadurch gekennzeichnet, daß man solche Hybridomzellen kloniert, deren Kulturüberstand solche Antikörper enthält, die mit dem Paraffin-behandelten c-kit-Rezeptor reagieren und die von diesen Klonen produzierten Antikörper nach bekannten Verfahren isoliert.

6. Verfahren zur Herstellung monoklonaler Antikörper gegen den humanen c-kit-Rezeptor nach Anspruch 1, welche die Stimulierung der Proliferation lymphoider Zellen durch SCF inhibieren durch Immunisierung mit Säugerzellen, die mit einer in diesen Zellen exprimierbaren c-kit-Sequenz transfiziert wurden, Immortalisierung der Milzzellen der immunisierten Tiere, Identifizierung derjenigen Hybridomzellen, die den gewünschten Antikörper produzieren, über die Bestimmung der Bindung des erhaltenen Antikörpers an den c-kit-Rezeptor, sowie die Bestimmung der Wirkung dieses Antikörpers auf die Proliferation lymphoider Zellen, Identifizierung solcher Hybridomzellen, deren Kulturüberstand dabei eine hemmende Wirkung aufweist, Klonierung dieser Hybridomzellen und Isolierung der von diesen Klonen produzierten Antikörper nach bekannten Verfahren.

7. Verfahren zur Herstellung monoklonaler Antikörper gegen den humanen c-kit-Rezeptor nach Anspruch 1, welche die SCF-induzierte Proliferation lymphoider Zellen stimulieren durch Immunisierung mit Säugerzellen, die mit einer in diesen Zellen exprimierbaren c-kit-Sequenz transfiziert wurden, Immortalisierung der Milzzellen der immunisierten Tiere, Identifizierung derjenigen Hybridomzellen, die den gewünschten Antikörper produzieren, über die Bestimmung der Bindung des erhaltenen Antikörpers an den c-kit-Rezeptor, sowie die Bestimmung der Wirkung dieses Antikörpers auf die SCF induzierte Proliferation lymphoider Zellen, Klonierung der auf diese Weise identifizierten gewünschten Hybridomzellen und Isolierung der von diesen Klonen produzierten Antikörpern nach bekannnten Verfahren.

8. Zellinie, die einen monoklonalen Antikörper gegen den humanen c-kit-Rezeptor produziert, ausgewählt aus der Gruppe DSM ACC 2007 und DSM ACC 2009.

9. Verfahren zum Nachweis der Malignität von Tumoren der hämatopoietischen Zellen, dadurch gekennzeichnet, daß man eine Gewebeprobe mit mindestens einem monoklonalen Antikörper gegen den c-kit-Rezeptor nach den Ansprüchen 1 - 3 inkubiert und anschließend gebundene Antikörper mittels bekannter Methoden nachweist.

10. Verfahren zum Nachweis der Malignität von Seminomen oder kleinzelligem Lungenkarzinom, dadurch gekennzeichnet, daß man eine Gewebeprobe mit mindestens einem monoklonalen Antikörper gegen den c-kit-Rezeptor inkubiert und anschließend gebundene Antikörper mittels bekannter Methoden nachweist.

## Claims

1. Monoclonal antibody against the human c-kit receptor obtainable from the cell lines DSM ACC 2007 or DSM ACC 2009 or which binds to the epitope to which the antibodies produced by the cell lines DSM ACC 2007 or DSM ACC 2009 bind.

2. Monoclonal antibody as claimed in claim 1 against the human c-kit receptor which inhibits the stimulation of the proliferation of lymphoid cells by SCF and which is obtainable by immunizing with mammalian cells which have been transfected with a c-kit sequence that can be expressed in these cells, immortalizing the spleen cells of the immunized animals, identifying those hybridoma cells which produce the desired antibody by determining the binding of the antibody obtained to the c-kit receptor as well as by determining the effect of this antibody on the proliferation of lymphoid cells, identifying those hybridoma cells whose culture supernatant has an inhibitory effect in this process, cloning these hybridoma cells and isolating the antibody produced by these clones according to known methods.

3. Monoclonal antibody as claimed in claim 1 against the human c-kit receptor which stimulates the SCF-induced proliferation of lymphoid cells and is obtainable by immunizing with mammalian cells which have been transfected with a c-kit sequence that can be expressed in these cells, immortalizing the spleen cells of the immunized animals, identifying those hybridoma cells which produce the desired antibody by determining the binding of the antibody obtained to the c-kit receptor as well as by determining the effect of this antibody on the proliferation of lymphoid cells, cloning the desired hybridoma cells identified in this way and isolating the antibody produced by these clones according to known methods.

4. Process for the production of monoclonal antibodies against the human c-kit receptor as claimed in claim 1 by immunizing with mammalian cells which have been transfected with a c-kit sequence that can be expressed in these cells, immortalizing the spleen cells of the immunized animals cloning those hybridoma cells which produce antibodies against the human c-kit receptor and isolating the antibodies produced by these clones according to known methods.

5. Process as claimed in claim 4, wherein those hybridoma cells are cloned whose culture supernatant contains those antibodies which react with the c-kit receptor treated with paraffin and the antibodies produced by these clones are isolated according to known methods.

6. Process for the production of monoclonal antibodies against the human c-kit receptor as claimed in claim 1 which inhibit the stimulation of the proliferation of lymphoid cells by SCF by immunizing with mammalian cells which have been transfected with a c-kit sequence that can be expressed in these cells, immortalizing the spleen cells of the immunized animals, identifying those hybridoma cells which produce the desired antibody by determining the binding of the antibody obtained to the c-kit receptor as well as by determining the effect of this antibody on the proliferation of lymphoid cells, identifying those hybridoma cells whose culture supernatant has an inhibitory effect in this process, cloning these hybridoma cells and isolating the antibodies produced by these clones according to known methods.

7. Process for the production of monoclonal antibodies against the human c-kit receptor as claimed in claim 1 which stimulate the SCF-induced proliferation of lymphoid cells by immunizing with mammalian cells which have been transfected with a c-kit sequence that can be expressed in these cells, immortalizing the spleen cells of the immunized animals, identifying those hybridoma cells which produce the desired antibody by determining the binding of the antibody obtained to the c-kit receptor as well as by determining the effect of this antibody on the SCF-induced proliferation of lymphoid cells, cloning the desired hybridoma cells identified in this manner and isolating the antibodies produced by these clones according to known methods.

8. Cell line which produces a monoclonal antibody against the human c-kit receptor selected from the group DSM ACC 2007 and DSM ACC 2009.

9. Method for testing the malignancy of tumours of haematopoietic cells, wherein a tissue sample is incubated with at least one monoclonal antibody against the c-kit receptor as claimed in claims 1 - 3 and subsequently bound antibodies are detected by means of known methods.

10. Method for testing the malignancy of seminomas or small-cell lung carcinoma, wherein a tissue sample is incubated with at least one monoclonal antibody against the c-kit receptor and subsequently bound antibodies are detected by means of known methods.

## Revendications

1. Anticorps monoclonal contre le récepteur c-kit humain, pouvant être obtenu à partir des lignées cellulaires DSM ACC 2007 ou DSM ACC 2009 ou qui se lie à l'épitope, auquel les anticorps produits à partir des lignées cellulaire DSM ACC 2007 ou DSM ACC 2009 se lient.

2. Anticorps monoclonal selon la revendication 1 contre le récepteur c-kit humain, lequel inhibe la stimulation de la prolifération de cellules lymphoïdes par et que l'on peut obtenir par une immunisation avec des cellules de mammifère, qui ont été transfectées avec une séquence c-kit pouvant être exprimée dans ces cellules, par immortalisation des cellules spléniques des animaux immunisés, par identification de ces hybridomes, qui produisent l'anticorps souhaité, en déterminant la liaison de l'anticorps obtenu au niveau du récepteur c-kit, ainsi qu'en déterminant l'effet de cet anticorps sur la prolifération de cellules lymphoïdes, par identification de tels hybridomes, dont l'excédent de culture présente en même temps un effet inhibiteur, par le clonage de ces hybridomes et isolation de l'anticorps produit par ces clones selon des procédés connus.

3. Anticorps monoclonal selon la revendication 1 contre le récepteur c-kit humain, lequel stimule la prolifération induite par SCF de cellules lymphoïdes et que l'on peut obtenir par immunisation avec des cellules de mammifère, qui ont été transfectées avec une séquence c-kit pouvant être exprimée dans ces cellules, par immortalisation des cellules spléniques des animaux immunisés, par identification de ces hybridomes, qui produisent l'anticorps souhaité, en déterminant la liaison de l'anticorps obtenu au niveau ou récepteur c-kit, ainsi qu'en déterminant l'effet de cet anticorps sur la prolifération de cellules lymphoïdes, par clonage des hybridomes souhaités identifiés de cette manière et par isolation de l'anticorps produit par ces clones selon des procédés connus.

4. Procédé pour la production d'anticorps monoclonaux contre le récepteur c-kit humain selon la revendication 1 par immunisation avec des cellules de mammifère, qui ont été transfectées avec une séquence c-kit pouvant être exprimée dans ces cellules, par immortalisation des cellules sphériques des animaux immunisés, par clonage de tels hybridomes, qui produisent des anticorps contre le récepteur c-kit humain et par isolation des anticorps produits par ces clones selon ces procédés connus.

5. Procédé selon la revendication 4, caractérisé en ce que l'on clone de tels hybridomes, dont l'excédent de culture contient de tels anticorps, qui réagissent avec le récepteur c-kit traité à la paraffine et que l'on isole les anticorps produits par ces clones selon des procédés connus.

6. Procédé pour la production d'anticorps monoclonaux contre le récepteur c-kit humain selon la revendication 1, lesquels inhibent la stimulation de la prolifération de cellules lymphoïdes par SCF car immunisation des cellules de mammifère, qui ont été transfectées avec une séquence c-kit pouvant être exprimée dans ces cellules, par immortalisation des cellules sphériques des animaux immunisés, par identification ce ces hybridomes qui produisent l'anticorps souhaité, en déterminant la liaison de l'anticorps obtenu au niveau du récepteur c-kit, ainsi qu'en déterminant l'effet de cet anticorps sur la prolifération de cellules lymphoïdes, par identification de tels hybridomes, dont l'excédent de culture présente en même temps un effet inhibiteur, par clonage de ces hybridomes et isolation des anticorps produits par ces clones selon des procédés connus.

7. Procédé pour la production d'anticorps monoclonaux contre le récepteur c-kit humain selon la revendication 1, lesquels stimulent la prolifération induite par SCF de cellules lymphoïdes par immunisation avec des cellules de mammifère, qui ont été transfectées avec une séquence c-kit pouvant être exprimée dans ces cellules, par immortalisation des cellules spléniques des animaux immunisés, par identification de ces hybridomes, qui produisent l'anticorps souhaité, en déterminant la liaison de l'anticorps obtenu au niveau du récepteur c-kit, ainsi qu'en déterminant l'effet de cet anticorps sur la prolifération induite par SCF de cellules lymphoïdes, par clonage des hybridomes souhaités identifiés de cette manière et par isolation des anticorps produits par ces clones selon des procédés connus.

8. Lignée de cellules qui produit un anticorps monoclonal contre le récepteur c-kit humain, choisie dans le groupe formé par DSM ACC 2007 et DSM ACC 2009.

9. Procédé pour détecter la malignité de tumeurs des cellules hématopoïétiques, caractérisé en ce que l'on incube un prélèvement tissulaire avec au moins un anticorps monoclonal contre le récepteur c-kit selon les revendications 1 à 3 et l'on détecte ensuite les anticorps liés au moyen de procédés connus.

10. Procédé pour détecter la malignité de séminomes ou de cancer du poumon à petites cellules, caractérisé en ce que l'on incube un prélèvement tissulaire avec au moins un anticorps monoclonal contre le récepteur c-kit et que l'on détecte ensuite les anticorps liés au moyen de procédés connus.
